# EUROPÄISCHE PATENTSCHRIFT

(11) **EP 2 694 527 B1**
(45) Veröffentlichungstag und Bekanntmachung des Hinweises auf die Patenterteilung: **18.01.2017**
(21) Anmeldenummer: 12712251.3
(22) Anmeldetag: 27.03.2012
(51) Int. Cl.: C07K 1/14, C12N 15/10, B01D 15/18, C07H 1/06, B01D 15/26, G01N 30/00

(54) **VERFAHREN ZUR TRENNUNG / REINIGUNG VON BIOMOLEKÜLEN**
PROCESS FOR SEPARATION/PURIFICATION OF BIOMOLECULES
PROCÉDÉ DE SÉPARATION/PURIFICATION DE BIOMOLÉCULES

(30) Priorität: 01.04.2011 DE 102011001743
(43) Veröffentlichungstag der Anmeldung: 12.02.2014
(73) Patentinhaber: Technische Universität Dortmund, 44227 Dortmund (DE)
(72) Erfinder: SCHEMBECKER, Gerhard, 44357 Dortmund (DE); BURGHOFF, Bernhard, 45219 Essen (DE); VAN WINSSEN, Fatma Alexia, 44229 Dortmund (DE)
(74) Vertreter: Michalski Hüttermann & Partner Patentanwälte mbB
(86) Internationale Anmeldenummer: PCT/EP2012/055434
(87) Internationale Veröffentlichungsnummer: WO 2012/130855

(56) Entgegenhaltungen:
- EP-A1- 1 609 800
- WO-A1-00/68260
- US-A- 5 882 520
- François Guyon ET AL: "Separation of sugars by h.p.l.c. on copper silicate gel", Carbohydrate Research, vol. 140, no. 1, 1 July 1985 (1985-07-01), pages 135-138, XP055193491, ISSN: 0008-6215, DOI: 10.1016/0008-6215(85)85057-6
- KABAY N ET AL: "Solvent-impregnated resins (SIRs) - Methods of preparation and their applications", REACTIVE & FUNCTIONAL POLYMERS, ELSEVIER SCIENCE PUBLISHERS BV, NL, vol. 70, no. 8, 1 August 2010 (2010-08-01) , pages 484-496, XP027124462, ISSN: 1381-5148 [retrieved on 2010-02-10]
- HANSSON U-B ET AL: "Fractionation of immunoglobulins by liquid-liquid partition chromatography in aqueous two-phase systems", ANALYTICAL BIOCHEMISTRY, ACADEMIC PRESS INC, NEW YORK, vol. 183, no. 2, 1 December 1989 (1989-12-01), pages 305-311, XP024820488, ISSN: 0003-2697, DOI: 10.1016/0003-2697(89)90484-3 [retrieved on 1989-12-01]
- KULA M R ET AL: "Investigations of liquid-liquid partition chromatography of proteins", JOURNAL OF CHROMATOGRAPHY, ELSEVIER SCIENCE PUBLISHERS B.V, NL, vol. 548, 12 July 1991 (1991-07-12), pages 3-12, XP026514485, ISSN: 0021-9673, DOI: 10.1016/S0021-9673(01)88589-7 [retrieved on 1991-07-12]
- Werner Muller: "New phase supports for liquid-liquid partition chromatography of biopolymers in aqueous poly(ethyleneglycol)-dextran systems. Synthesis and application for the fractionation of DNA restriction fragments", European Journal of Biochemistry, vol. 155, no. 1, 1 February 1986 (1986-02-01), pages 213-222, XP055193517, ISSN: 0014-2956, DOI: 10.1111/j.1432-1033.1986.tb09479.x
- DIAMOND A D ET AL: "AQUEOUS TWO-PHASE SYSTEMS FOR BIOMOLECULE SEPARATION", ADVANCES IN BIOCHEMICAL ENGINEERING, BIOTECHNOLOGY, SPRINGER, BERLIN, DE, vol. 47, 1 January 1992 (1992-01-01), pages 89-135, XP002930072, ISSN: 0724-6145
- ALPERT ET AL: "Hydrophilic-interaction chromatography for the separation of peptides, nucleic acids and other polar compounds", JOURNAL OF CHROMATOGRAPHY, ELSEVIER SCIENCE PUBLISHERS B.V, NL, vol. 499, 19 January 1990 (1990-01-19), pages 177-196, XP026484536, ISSN: 0021-9673, DOI: 10.1016/S0021-9673(00)96972-3 [retrieved on 1990-01-19]

## Beschreibung

Die vorliegende Erfindung bezieht sich auf das Gebiet der Trennung bzw. Reinigung von Biomolekülen, insbesondere auf das Gebiet der Trennung bzw. Reinigung von Biomolekülen mittels wässriger Zweiphasen-Extraktion.

Die wässrige Zweiphasen-Extraktion (engl. Aequous Two-Phase Extraction, ATPE) ist eine auf dem Gebiet der Analytik und Gewinnung von Biomolekülen weitverbreitete, jedoch bisher nicht großtechnisch eingesetzte Trennungsmethode. Dies liegt im Wesentlichen an der häufig nur sehr langsamen Phasentrennung der beiden wässrigen Phasen sowie an dem häufigen Phänomen, dass sich durch die Anwesenheit der Biomoleküle persistente Emulsionen bilden, die nur durch (starke) Zentrifugation getrennt werden können.

Trennverfahren sind u.a. aus der EP 1 609 800, der WO 00/68260 und der US 5,882,520, sowie Kula et al, J. of Chromatography, 1991, 3, Müller, Eur. J. Biochemistry, 1986, 219, sowie Diamond et al, Advances in Biochemical Engineering, 1992, 89 bekannt.

Es stellt sich somit die Aufgabe, ein verbessertes Zweiphasen-Extraktionsverfahren zu schaffen.

Diese Aufgabe wird durch ein Verfahren gemäß Anspruch 1 der vorliegenden Erfindung gelöst. Demgemäß wird ein Verfahren zur Trennung bzw. Reinigung von Biomolekülen durch wässrige Zweiphasen-Extraktion mittels einer ersten wässrigen Phase und einer zweiten wässrigen Phase vorgeschlagen, wobei während der Trennung zumindest partielle Überführung der Biomoleküle in die zweite wässrige Phase erfolgt, wobei ein poröses Material vorhanden ist, in dessen Poren zweite wässrige Phase vorhanden ist und während der Trennung eine Lösung von Biomolekülen in der in den Poren des porösen Materials vorhandenen zweiten wässrigen Phase erfolgt.

Unter der Bezeichnung "Trennung" werden insbesondere alle Verfahren verstanden, bei denen
- Biomoleküle aus Fermentationsbrühen in Reinform durch Lösung in der zweiten wässrigen Phase isoliert werden (Capture)
- Gruppen von zwei oder mehr der vorgenannten Biomoleküle aus dem Fermentationsmedium abgetrennt werden
- Biomoleküle aus den bei der Biokatalyse anfallenden flüssigen Medien in Reinform isoliert werden (Capture)
- Gruppen von zwei oder mehr der vorgenannten Biomoleküle aus Biokatalysemedien abgetrennt werden
- Unerwünschte Nebenkomponenten, wie z.B. alle Arten von unerwünschten Proteinen (Proteasen, Membranproteine, etc.), Viren, DNA, Zellorganellen (Mitochondrien, etc.) und andere Zelltrümmer, Lipide, Metaboliten und Stoffwechselprodukte aus Fermentationsbrühen einzeln oder in Gruppen entfernt werden
- Unerwünschte Nebenkomponenten aus den bei der Biokatalyse anfallenden flüssigen Medien einzeln oder in Gruppen entfernt werden

Unter der Bezeichnung "Reinigung" werden insbesondere alle Verfahren verstanden, bei denen
- Biomoleküle aus Fermentationsbrühen in Reinform durch Lösung in der zweiten wässrigen Phase isoliert werden (Polishing)
- Biomoleküle aus den bei der Biokatalyse anfallenden flüssigen Medien in Reinform durch Lösung in der zweiten wässrigen Phase isoliert werden (Polishing)

Unter dem Term "Biomoleküle" werden Nukleinsäuren, Antibiotika, Aminosäuren, Lipide, Kohlenhydrate, Metabolite, Inclusion Bodies, Stoffwechselprodukte und insbesondere alle Arten von Proteinen verstanden, darunter (aber nicht darauf beschränkt)
- Transportproteine
- Antikörper
- Membranproteine
- Hormone
- Blutgerinnungsfaktoren
- Enzyme
- Kollagene
- Endotoxine

Unter dem Term "poröses Material" werden insbesondere - aber nicht darauf beschränkt - alle Materialien verstanden, die über eine offene Porosität von ≥50% verfügen. Überraschenderweise hat sich gezeigt, dass durch ein solches poröses Material die wässrige Zweiphasen-Extraktion wesentlich verbessert werden kann. Für viele Anwendungen innerhalb der vorliegenden Erfindung erfüllt das erfindungsgemäße Verfahren mindestens einen oder mehrere der folgenden Vorteile:
- Dadurch, dass zweite wässrige Phase in dem porösen Material enthalten ist, kann eine Abtrennung der Biomoleküle, die nach erfolgter Trennung sich in dieser wässrigen Phase (und somit "innerhalb" des porösen Materials) aufhalten, auf einfache Weise erfolgen.
- Durch die Vermeidung langwieriger Phasentrennung kommt es zu erhelblicher Zeitersparnis; auf meist notwendige separate Zentrifugationsschritte kann - zusammen mit dem damit verbundenen apparativen Aufwand - verzichtet werden
- Der Raumbedarf im Vergleich zur wässrigen Zweiphasenextraktion sinkt, da der Zentrifugationsschritt eingespart wird; hierdurch kommt es neben der Raumersparnis zu einer Investitionskostenersparnis
- Einfaches Scale-Up durch Anwendung der in Adsorption und Chromatographie bekannten Heuristiken und Rechenmodelle

Gemäß einer bevorzugten Ausführungsform der Erfindung beträgt die durchschnittliche Porengröße des porösen Materials von ≥0,1 nm bis ≤5000 nm. Dies hat sich in der Praxis bewährt. Besonders bevorzugt beträgt die durchschnittliche Porengröße des porösen Materials von ≥2 nm bis ≤2000 nm, noch bevorzugt von ≥50 nm bis ≤1000 nm.

Der Term "durchschnittlich" bedeutet dabei insbesondere, dass es sich um den mathematisch gemittelten Wert der gesamten über ein Partikel bzw. eine Partikelcharge produktionsbedingt (in der Regel) gemäß einer Gaußverteilung gestreuten Durchmesser der Poren handelt. Da die Bestimmung der Porendurchmesser häufig unter Zuhilfenahme bildgebender Verfahren durchgeführt wird, wird unter Porendurchmesser im Sinne der vorliegenden Erfindung insbesondere die auf der Partikeloberfläche sichtbaren Durchmesser der Poren verstanden.

Wie sich in der Praxis bei vielen Anwendungen gezeigt hat, bedeutet eine hohe Porosität aufgrund großen konstanten Porendurchmessers bzw. sich ins Partikelinnere erweiternder Poren bzw. eines im Partikel vorhandenen zentralen Hohlraumes eine hohe Kapazität für die Aufnahme zweiter wässriger Phase und somit potentiell eine höhere Kapazität für die abzutrennende(n) Zielkomponente(n) als im Vergleich zu Partikeln mit geringerem konstanten Porendurchmesser bzw. sich im Querschnitt ins Partikelinnere verengenden Poren bzw. ohne zentralen Hohlraum.

Gemäß einer bevorzugten Ausführungsform der Erfindung ist während der Durchführung der Trennung bzw. Reinigung die zweite wässrige Phase im Wesentlichen in den Poren des porösen Materials vorhanden.

"Im Wesentlichen" im Sinne der vorliegenden Erfindung bedeutet dabei ≥90 Gew-%, bevorzugt ≥95 Gew-%, noch bevorzugt ≥97 Gew-%, sowie am meisten bevorzugt ≥99 Gew-%.

Dies hat sich als vorteilhaft herausgestellt, da so nach der durchgeführten Trennung die in der zweiten wässrigen Phase befindlichen Biomoleküle sich auch innerhalb des porösen Materials aufhalten. Jedoch sei ausdrücklich darauf hingewiesen, dass das vorliegende Verfahren nicht darauf beschränkt ist, je nach konkreter Ausgestaltung kann es auch von Vorteil sein, dass sich die zweite wässrige Phase auch ausserhalb des porösen Materials befindet, dieses sozusagen eher "unterstützend" eingesetzt wird.

Gemäß der Erfindung befindet sich vor und nach der Trennung die erste wässrige Phase im Wesentlichen außerhalb des porösen Materials. Dies hat sich als vorteilhaft herausgestellt, da so die Trennung der Phasen erleichtert wird. Gemäß einer bevorzugten Ausführungsform der Erfindung handelt es sich bei dem porösen Material im Wesentlichen um ein partikelförmiges Material. Dies hat sich bei vielen Anwendungen als besonders vorteilhaft herausgestellt, da dadurch oftmals einer oder mehrerer der folgenden Vorteile erreicht werden können:
- Die Partikel können in eine Säule gepackt und somit als präparatives "quasi-chromatographisches" Festbett verwendet werden
- Da die Poren der Partikel als "Hohlraum" für die zweite wässrige Phase dienen können die Partikel leicht wiederverwertet werden, zudem ist eine schnelle Anpassung/Umstellung möglich, sowie eine GMP konforme (Re-)Imprägnierung in geschlossenen Apparaten.

Gemäß einer bevorzugten Ausführungsform beträgt die durchschnittliche Partikelgröße des porösen Materials von ≥0,01 mm und ≤20 mm, bevorzugt ≥0,05 mm und ≤15 mm sowie am meisten bevorzugt ≥0,1 mm und ≤5 mm, bevorzugt ≤2 mm.

Gemäß einer bevorzugten Ausführungsform der Erfindung erfolgt während der Trennung eine Adsorption von Biomolekülen an das poröse Material. Dies ist oftmals ein willkommener "Nebeneffekt" bei der Verwendung von bestimmten porösen Materialien.

Gemäß einer bevorzugten Ausführungsform der Erfindung hat das poröse Material eine Schwellrate von ≤40%. Dies hat sich in der Praxis als vorteilhaft herausgestellt, da so kein "Lecken" der in dem porösen Materials befindlichen zweiten wäßrigen Phase erfolgt. Für viele Anwendungen ist jedoch eine gewisse Schwellrate vorteilhaft, da so die Kapazität für die zweite wässrige Phase steigt. Gemäß einer bevorzugten Ausführungsform der Erfindung hat das poröse Material eine Schwellrate von ≥20% und ≤35%.

Gemäß einer bevorzugten Ausführungsform der Erfindung ist das poröse Material im Wesentlichen ausgewählt aus der Gruppe enthaltend:
- Polymerpartikel auf Basis von Polystyrol-Divinylbenzol, Polypropylen, Melamin, Poly(methyl methacrylat), Polyvinylacetat, Polyvinylchlorid, Polystyrol, Polyacrylamid, Polybutylacrylat; diese Polymere ggf. in amorpher, semi-kristalliner und kristalliner Form
- Magnetische (Polymer)-Partikel
- Mikrokapseln
- Kohlenstoffhaltige Polymerpartikel, sog. Carbonaceous Resins
- Beschichtete Polymerpartikel, sog. Coated Particles, wobei die Beschichtung u.a. durch Polyvinylalkohol erreicht werden kann
- Polymere Anionen- und Kationenaustauscher
- Zeolite
- Silica / Silicate
- Aktivkohle
- Anorganische Anionen- und Kationenaustauscher
- Metal Organic Frameworks
- Glas
- Carbonate
- Polymerschäume
- Metall schäume
oder Mischungen daraus.

Gemäß einer bevorzugten Ausführungsform wird als wässrige Zweiphasen-Extraktion die polymere Zweiphasen-Extraktion (ATPPE), die mizellare Zweiphasen-Extraktion (ATPME), die reverse mizellare Zweiphasen-Extraktion (ATPRME) und/oder die Zweiphasen-Extraktion unter der Verwendung ionischer Flüssigkeiten verwendet.

Im Folgenden wird auf die einzelnen Extraktionsverfahren genauer eingegangen:

### Polymere Zweiphasen-Extraktion (ATPPE)

Für den Fall, dass die polymere Zweiphasen-Extraktion (ATPPE) zum Einsatz kommt, sind insbesondere folgende Spezifikationen besonders bevorzugt:
- Erste und zweite wässrige Phase enthalten nichtionische Polymere

In diesem Fall sind folgende Systeme besonders bevorzugt:
- Polyoxyverbindungen wie Polyethylenglykol/Polypropylenglykol einerseits, Polysaccharide wie z.B. Dextran andererseits
- Polyoxyverbindungen wie Polyethylenglykol/Polypropylenglykol einerseits, Polyhydroxypolymere wie Polyvinylalkohol andererseits
- Ein nichtionisches Polymer und ein Polyelektrolyt

In diesem Fall sind folgende Systeme besonders bevorzugt:
- Polyoxyverbindungen wie Polyethylenglykol/Polypropylenglykol einerseits, sulfonierte Polysaccharide wie z.B. Natriumdextransulfat andererseits
- Polyoxyverbindungen wie Polyethylenglykol/Polypropylenglykol einerseits, Polyimine wie Polyethylenimin andererseits
- modifizierte Polysaccharide wie Methylcellulose einserseits, sulfonierte Polysaccharide wie z.B. Natriumdextransulfat andererseits
- Erste und zweite wässrige Phase enthalten Elektrolyte

In diesem Fall sind folgende Systeme besonders bevorzugt:
- sulfonierte Polysaccharide wie z.B. Natriumdextransulfat einerseits, modifizierte Polysaccharide wie Natriumcarboxydextran andererseits
- modifizierte Polysaccharide wie Natriumcarboxycellulose einserseits, anders modifizierte Polysaccharide wie Natriumcarboxydextran andererseits
- Ein nichtionisches Polymer und eine hochkonzentrierte Lösung eines niedermolekularen Stoffes

In diesem Fall sind folgende Systeme besonders bevorzugt:
- Polyoxyverbindungen wie Polyethylenglykol/Polypropylenglykol einerseits, niedermolekulare Saccharide wie Glucose andererseits
- Polyoxyverbindungen wie Polyethylenglykol/Polypropylenglykol einerseits, niedermolekulare Salze wie Citrat oder Phosphat andererseits.

Die oben genannten Polymere und Polyelektrolyten können zur weiteren Verbesserung der Trennleistung mit Affinitätsliganden gekoppelt werden, z.B. glutarsäure-, amino-, mercaptoethylpyridin-, pyrimidin-, benzyl- and triazin-basierte Gruppen.

### Mizellare Zweiphasen-Extraktion (ATOME)

Für den Fall, dass die mizellare Zweiphasen-Extraktion (ATPME) zum Einsatz kommt, sind insbesondere folgende Spezifikationen besonders bevorzugt:
- Das System enthält nichtionische Tenside

In diesem Fall sind folgende Systeme besonders bevorzugt:
- Eine wässrige Lösung bestehend aus polyethylenoxidbasierten nichtionischen Tensiden wie n-Decyltetraethylenoxid (C10E4) und Octylphenolethoxylat (Triton X-114), die sich in Abhängigkeit von der Temperatur in eine mizellenreiche Phase und eine mizellenarme Phase trennen lässt
- Das System enthält nichtionische und kationische Tenside

In diesem Fall sind folgende Systeme besonders bevorzugt:
- Eine wässrige Lösung bestehend aus polyethylenoxidbasierten nichtionischen Tensiden wie n-Decyltetraethylenoxid (C10E4) und kationischen Tensiden wie Alkyltrimethylammoniumbromid, die sich in Abhängigkeit von der Temperatur in eine mizellenreiche Phase und eine mizellenarme Phase trennen lässt
- Das System enthält eine Mischung anionischer und kationischer Tenside

In diesem Fall sind folgende Systeme besonders bevorzugt:
- Eine wässrige Lösung bestehend aus anionischen Tensiden wie Dodecyltriammoniumbromid (C12NE) und kationischen Tensiden wie Natriumperfluorooctanoat (SPFO)

### Reverse mizellare Zweiphasen-Extraktion (ATPRME)

Für den Fall, dass die reverse mizellare Zweiphasen-Extraktion (ATPRME) zum Einsatz kommt, sind insbesondere folgende Spezifikationen besonders bevorzugt:
- Erste Phase enthält kationische Tenside und zweite Phase enthält organische Lösungsmittel

In diesem Fall sind folgende Systeme besonders bevorzugt:
- n-Benzyl-n-dodecyl-n-bis(2-hydroxyethyl)ammoniumchlorid (BDBAC)/Isooctan/Hexanol
- Cetyltrimethylammoniumbromid (CTAB)/Isooktan/HexanolButanol

### Zweiphasen-Extraktion unter der Verwendung ionischer Flüssigkeiten

Für den Fall, dass die Zweiphasen-Extraktion unter der Verwendung ionischer Flüssigkeiten zum Einsatz kommt, sind insbesondere folgende Spezifikationen besonders bevorzugt:
- Erste wässrige Phase enthält ionische Flüssigkeit und zweite wässrige Phase enthält Elektrolyte

In diesem Fall sind folgende Systeme besonders bevorzugt:
- Ionische Flüssigkeiten wie Ammoeng™ 110 einerseits, Salze wie Kaliumhydrogenphosphat (K2HPO4/KH2PO4) andererseits
- Ionische Flüssigkeiten wie 1-butyl-3-methylimidazoliumchlorid ([Bmim][Cl]), Salze wie Kaliumhydrogenphosphat (K2HPO4) andererseits
- Ionische Flüssigkeiten wie 1-butyl-3-methylimidazoliumtertafluoroborat ([Bmim][BF4]), Salze wie Natriumhydrogenphosphat (NaH2PO4) andererseits

Gemäß einer bevorzugten Ausführungsform der Erfindung umfasst das erfindungsgemäße Verfahren zur Trennung bzw. Reinigung von Biomolekülen folgende Schritte
a) "Befüllen" bzw. "Imprägnieren" des porösen Materials mit der zweiten wässrigen Phase
b) Suspendieren des porösen Materials in der ersten wässrigen Phase
c) Hinzugeben der die zu trennenden bzw. reinigenden Biomoleküle enthaltenden Mischung
d) Durchführung der Trennung
e) Abtrennen des porösen Materials
f) Gewinnen der abgetrennten Biomoleküle aus der in den Poren des porösen Materials enthaltenden zweiten wässrigen Phase

Gemäß einer bevorzugten Ausführungsform wird die zweite wässrige Phase vor oder während Schritt a) mit der ersten wässrigen Phase gesättigt. Dies hat den Vorteil, dass während der Trennung keine (oder nur sehr wenig) erste wässrige in die Poren des porösen Materials eindringt.

Für den Fachmann ist einsichtig, dass die Schritte b) und c) in beliebiger Reihenfolge bzw. auch simultan erfolgen können. Bei einigen Ausführungen der Erfindung geschieht die Abtrennung der gewünschten Biomoleküle auch direkt aus der "Reaktionslösung" (z.B. bei Fermenterprozessen), d.h. die erste wässrige Phase und die zu trennenden bzw. reinigenden Biomoleküle enthaltende Mischung sind (teil)-identisch.

Schritt d) kann entweder durch reines Abwarten (d.h. durch Diffusion) erfolgen, meist wird aber eine aktive Durchmischung erfolgen, diese kann z.B. durch Rühren z.B. mittels eines Impellers, durch Anströmung im Festbett ähnlich chromatographischer Methoden oder durch Dispersion im Wirbelbett mittels Durchströmung einer losen Schüttung geschehen.

Schritt e) kann im einfachsten Fall dadurch geschehen, dass das poröse Material abfiltriert wird.
Die Filtration kann durch dynamische Filtration (z.B. Cross-Flow-Filtration) sowie durch statische Filtration (z.B. Druck- oder Membranfiltration) erreicht werden.
Bei Verwendung eines magnetischen porösen Materials kann die Abtrennung des porösen Materials durch Anlegen eines (elektro-)magnetischen Feldes erzielt werden. Des Weiteren kann die Abtrennung des porösen Materials durch gravimetrische Setzung erreicht werden. Zudem kann zur Abtrennung des porösen Materials eine Zentrifuge bzw. ein Dekanter verwendet werden.

Schritt f) kann z.B. durch Rückextraktion der Biomoleküle aus der zweiten Phase mittels konzentrierter Salzlösungen bzw. durch Änderung der Prozessparameter, wie z.B. pH-Wert, Ionenstärke, Zugabe eines zusätzlichen Salzes geschehen.

Eine weitere Ausführungsform der Erfindung bezieht sich auf eine Verwendung eines erfindungsgemäßen Verfahrens zur
- großtechnischen Gewinnung von Proteinen
- großtechnischen Gewinnung von Antibiotika, Aminosäuren, (Oligo-)Peptiden, Nukleinsäuren, Lipiden, Kohlenhydraten, Metaboliten, Stoffwechselprodukten, Inclusion Bodies, Plasmiden
- analytischen Trennung von Biomolekülen
- großtechnischen Abtrennung unerwünschter Komponenten wie Viren, Proteasen, Zelltrümmer, etc. aus Biomolekülen enthaltenden Medien zwecks Vorreinigung der Biomoleküle enthaltenden Medien
- Phytochemie

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus den nachfolgenden Beschreibungen der zugehörigen Beispiele, die rein illustrativ zu verstehen sind.

### Beispiel I: Abtrennung von BSA-Protein

Folgende Spezifikationen wurden gewählt:
- Erste wässrige Phase: 20% w/w Trinatriumcitratlösung
- Zweite wässrige Phase: 20% (w/w) Polyethylenglykollösung
- Poröses Material: Amberlite XAD16- Partikel (Quervernetztes Polystyrol-Divinylbenzol, Porosität 0,55, Porengröße 10 nm, Partikelgröße 0,56-0,71 mm)
- Abzutrennende/reinigende Biomoleküle: BSA (Bovine Serum Albumin)- Protein a) "Befüllen" bzw. Imprägnieren des porösen Materials.

Hierzu wurde im Wesentlichen die Methode nach J.L. Cortina und A. Warshawsky, Developments in solid-liquid extraction by solvent-impregnated-resins, Ion Exch. Solvent Extr. 13, 195-293, 1997 verwendet: Zunächst wurde die zweite wässrige Phase mit erster wässriger Phase voräquilibriert. Anschließend wurden 2g poröses Material in der zweiten wässrigen Phase komplett dispergiert. Die Dispersion wurde für die Dauer von 30 min. in ein Ultraschallbad gestellt, anschließend wurde filtriert und die an den Partikeln haftenden Überreste an wässriger Phase mit Zellstoff entfernt.

### b) Abtrennung der Biomoleküle

Im zweiten Schritt wurde die erste wässrige Phase mit BSA-Proteinlösung versetzt, so dass sich eine Konzentration von 0,5mg / ml Protein ergab. Anschließend wurden 2g des porösen Materials mit 10ml erster wässriger Phase versetzt und 180min. durch Schütteln mit einem Überkopfschüttler dispergiert. Anschließend ließ man zentrifugieren. Aus dem Überstand wurde eine Probe entnommen und die verbliebene Proteinkonzentration bestimmt.

### c) Vergleichsversuche

Parallel wurde eine Zweiphasenextraktion ohne Zusatz von porösem Material durchgeführt; hierzu wurden 5ml der voräquilibrierten zweiten Phase mit 10ml der proteinhaltigen ersten Phase versetzt und unter Rühren für 180min. emulgiert. Nach erfolgter Phasentrennung wurde die verbliebene Proteinkonzentration in der ersten wässrigen Phase bestimmt.

Als weiterer Vergleichsversuch wurde die Adsorption des Proteins an das poröse Material bestimmt; hierzu wurde eine 0,5 mg/ml Proteinlösung mit porösem Material versetzt; man ließ (analog zu Schritt b) dispergieren und bestimmte anschließend die verbliebene Proteinkonzentration.

Die Versuchsergebnisse sind in Tabelle I aufgeführt:

| Methode | Abreicherung des Proteins (in %) |
|---|---|
| Erfindungsgemäße Methode | 22,22 % |
| Zweiphasenextraktion ohne poröses Material | 12,26 % |
| Adsorption aus Wasser | 8,65 % |

Man sieht somit klar, dass - neben der schnelleren Durchführbarkeit des erfindungsgemäßen Verfahrens - auch die Abreicherung des Proteins deutlich gesteigert werden konnte.

### d) Untersuchung des porösen Materials

Um den Grad der Adsorption des Proteins an das poröse Material (im Unterschied zur Lösung in der zweiten wässrigen Phase, die sich in den Poren befindet) festzustellen wurde folgende Untersuchung vorgenommen.

Das die zweite Phase (PEG-Lösung) enthaltende poröse Material wurde mittels einer Vakuumpumpe und Filterpapier von dem das Zielprotein enthaltenden Überstand der ersten Phase (Salzlösung) getrennt. Danach werden auf das poröse Material 20 mL der mit der ersten Phase (Salzlösung) vorgesättigten zweiten Phase (PEG-Lösung) gegeben. Diese Dispersion wird für 180 min. im Überkopfschüttler bei 10 rpm vermischt. Hiernach wurde der Überstand der das poröse Material umgebenden PEG-Lösung mittels einer Pipette abgenommen, zenrtifugiert (5000 rpm) und sterilfiltriert. Von der filtrierten PEG-Lösung wurden 5 mL zwecks Rückextraktion des Proteins mit 5 mL (mit PEG-Lösung) vorgesättigter Salzlösung für 180 min im Überkopfschüttler emulgiert. Im Anschluss daran wurden die Phasen durch Zentrifugation (1500 rpm) getrennt und eine Probe der Salzlösung entnommen. Diese wurde mittels eines UV/VIS-Spektrophotometers bei 280 nm auf ihren Proteingehalt untersucht. Diese Messungen haben ergeben, dass mindestens ca. 50% des Proteins in gelöster Form, d.h. nicht adsorbiert, innerhalb der in den Poren des porösen Materials vorhandenen zweiten Phase vorhanden ist.

### Beispiel II

Folgende Spezifikationen wurden gewählt:
- Erste wässrige Phase: 20% w/w Dikaliumhydrogenphosphatlösung
- Zweite wässrige Phase: 20% (w/w) Polyethylenglykollösung
- Poröses Material: Amberlite XAD16- Partikel
- Abzutrennende/reinigende Biomoleküle: BSA (Bovine Serum Albumin)- Protein

Die Vorgehensweise entsprach Beispiel I. Die Versuchsergebnisse sind in Tabelle II aufgeführt:

| Methode | Abreicherung des Proteins (in %) |
|---|---|
| Erfindungsgemäße Methode | 32,03% |
| Zweiphasenextraktion ohne poröses Material | 15,48% |
| Adsorption aus Wasser | 8,65 % |

### Beispiele III bis V

Die Spezifikationen der Beispiele III und V entsprechen denen aus Beispiel I, nur das jeweils ein anderes poröses Material gewählt wurde. Die Versuchsergebnisse sind in Tabelle III aufgeführt.

| Beispiel | Abreicherung des Proteins (in %) |
|---|---|
| Beispiel III: Lewatit VPOC 1064 | 42,55% |
| Beispiel IV: Amberlite XAD18 | 48,85% |
| Beispiel V: Lewatit CNP 105 | 95,11 % |

Die Eigenschaften der verwendeten porösen Materialien sind in Tabelle IV zusammengefasst.

| Material | Chemische Zusammensetzung | Porengröße (in nm) | Porenvolumen (in mg/L) | Partikeldurchmesser (in mm) |
|---|---|---|---|---|
| Lewatit VPOC 1064 | Quervernetztes Polystyrol | 5 - 10 | 1,02 | 0,44 - 0,54 |
| Amberlite XAD18 | Quervernetztes Polystyrol-Divinylbenzol | 15 | k.A. | 0,375 - 0,475 |
| Lewatit CNP 105 | Methacrylat-Polymer | ∼27 | k.A. | 0,1 - 0,4 |

### Beispiele VI bis XI

Die Spezifikationen und Ergebnisse sind in Tabelle V bzw. VI zusammengefasst

| Beispiel | Protein | Zweiphasensystem | Partikel |
|---|---|---|---|
| VI | Lysozym | PEG2000/NaCitrat (20 Gew.-%/20 Gew.-%) | Amberlite XAD16 |
| VII | Myoglobin | PEG2000/NaCitrat (20 Gew.-%/20 Gew.-%) | Amberlite XAD16 |
| VIII | Bovine Serum Albumin | PEG2000/NaCitrat (20 Gew.-%/20 Gew.-%) | Amberlite XAD16 |
| IX | Alpha-Lactalbumin | PEG2000/NaCitrat (20 Gew.-%/20 Gew.-%) | Amberlite XAD16 |
| X | Cytochrom C | PEG2000/NaCitrat (20 Gew.-%/20 Gew.-%) | Amberlite XAD16 |
| XI | Bovine Serum Albumin | PEG4000/ Dx500000 (Dextran) (8 Gew.-%/12 Gew.-%) | Stuttgarter Masse 3-5 (Fa. Pall) |

| Beispiel | Abreicherung des Proteins (Zweiphasen-Extraktion) in % | Abreicherung des Proteins (Erfindungsgemäße Methode) in % | Faktor |
|---|---|---|---|
| VI | 32,47 | 86,80 | 2,67 |
| VII | 12,53 | 66,73 | 5,33 |
| VIII | 9,63 | 41,69 | 4,33 |
| IX | 6,10 | 12,86 | 2,11 |
| X | 3,08 | 6,37 | 2,06 |
| XI | 0,63 | 0,73 | 1,16 |

## Patentansprüche

1. Verfahren zur Trennung bzw. Reinigung von Biomolekülen durch wässrige Zweiphasen-Extraktion mittels einer ersten wässrigen Phase und einer zweiten wässrigen Phase, wobei während der Trennung zumindest partielle Überführung der Biomoleküle in die zweite wässrige Phase erfolgt, wobei ein poröses Material vorhanden ist, in dessen Poren zweite wässrige Phase vorhanden ist und während der Trennung eine Lösung von Biomolekülen in der in den Poren des porösen Materials vorhandenen zweiten wässrigen Phase erfolgt, und wobei es sich bei dem porösen Material um ein partikelförmiges Material handelt wobei während der Durchführung der Trennung bzw. Reinigung die zweite wässrige Phase im Wesentlichen in den Poren des porösen Materials vorhanden ist sowie vor und nach der Trennung sich die erste wässrige Phase im Wesentlichen außerhalb des porösen Materials befindet. und wobei die Biomoleküle ausgewählt sind aus Nukleinsäuren, Antibiotika, Aminosäuren, Lipide, Kohlenhydrate, Metabolite, Inclusion Bodies, Stoffwechselprodukte und alle Arten von Proteinen.

2. Verfahren nach Anspruch 1, wobei die durchschnittliche Porengröße des porösen Materials von ≥0,1 nm bis ≤5000 nm beträgt

3. Verfahren nach Anspruch 1 oder 2, wobei die durchschnittliche Partikelgröße des porösen Materials ≥0,01 mm und ≤20 mm beträgt

4. Verfahren nach einem der Ansprüche 1 bis 3, wobei das poröse Material eine Schwellrate von ≤40% aufweist

5. Verfahren nach einem der Ansprüche 1 bis 4, wobei das poröse Material im Wesentlichen ausgewählt ist aus der Gruppe enthaltend
- Polymerpartikel auf Basis von Polystyrol-Divinylbenzol, Polypropylen, Melamin, Poly(methyl methacrylat), Polyvinylacetat, Polyvinylchlorid, Polystyrol, Polyacrylamid, Polybutylacrylat; diese Polymere ggf. in amorpher, semi-kristalliner und kristalliner Form
- Magnetische (Polymer)-Partikel
- Mikrokapseln
- Kohlenstoffhaltige Polymerpartikel, sog. Carbonaceous Resins
- Beschichtete Polymerpartikel, sog. Coated Particles, wobei die Beschichtung u.a. durch Polyvinylalkohol erreicht werden kann
- Polymere Anionen- und Kationenaustauscher
- Zeolite
- Silica / Silicate
- Aktivkohle
- Anorganische Anionen- und Kationenaustauscher
- Metal Organic Frameworks
- Glas
- Carbonate
- Polymerschäume
- Metallschäume
oder Mischungen daraus.

6. Verwendung eines Verfahrens gemäß der Ansprüche 1 bis 5 zur
- großtechnischen Gewinnung von Proteinen
- großtechnischen Gewinnung von Antibiotika, Aminosäuren, (Oligo-)Peptiden, Nukleinsäuren, Lipiden, Kohlenhydraten, Metaboliten, Stoffwechselprodukten, Inclusion Bodies, Plasmiden
- analytischen Trennung von Biomolekülen
- großtechnischen Abtrennung unerwünschter Komponenten wie Viren, Proteasen, Zelltrümmer, etc. aus Biomolekülen enthaltenden Medien zwecks Vorreinigung der Biomoleküle enthaltenden Medien
- Phytochemie

## Claims

1. Method for separating or purifying biomolecules by aqueous two-phase extraction by means of a first aqueous phase and a second aqueous phase, wherein during the separation at least a partial transition of the biomolecules into the second aqueous phase occurs, wherein a porous material is present, in which pores the second aqueous phase is present and wherein during the separation a solution of biomolecules in the second aqueous phase present in the pores of the porous material occurs, and wherein the porous material is a particulate material,
wherein during the implementation of the separation or purification the second aqueous phase is present substantially in the pores of the porous material and prior and subsequently to the separation the first aqueous phase is substantially disposed outside the porous material, and
wherein the biomolecules are selected from nucleic acids, antibiotics, amino acids, lipids, carbohydrates, metabolites, metabolites, inclusion bodies, metabolic products and all kinds of proteins.

2. Method according to claim 1, wherein the average pore size of the porous material is in the range of ≥ 0,1 nm to ≤ 5000 nm.

3. Method according to claim 1 or 2, wherein the average particle size of the porous material is in the range of ≥ 0,01 nm to ≤ 20 nm.

4. Method according to any one of claims 1 to 3, wherein the porous material has a swelling rate of ≤ 40%.

5. Method according to any one of claims 1 to 4, wherein the porous material is substantially selected from the group consisting of
- polymer particles based on polystyrene-divinylbenzene, polypropylene, melamine, poly(methyl methacrylate), polyvinyl acetate, polyvinyl chloride, polystyrene, polyacrylamide, polybutylacrylate, optionally in amorphous, semi-crystalline and crystalline form;
- magnetic (polymer) particles;
- microcapsules;
- carbon-containing polymer particles, so-called carbonaceous resins;
- coated polymer particles, so-called coated particles, wherein the coating can be achieved, inter alia, by polyvinyl alcohol;
- polymeric anion and cation exchangers;
- zeolites;
- silica/silicates;
- activated carbon;
- inorganic anion and cation exchangers;
- metal organic frameworks;
- glass;
- carbonates;
- polymer foams;
- metal foams;
or mixtures thereof.

6. Use of a method according to any one of claims 1 to 5 for the
- production of proteins on an industrial scale;
- production of antibiotics, amino acids, (oligo) peptides, nucleic acids, lipids, carbohydrates, metabolites, metabolic products, inclusion bodies, plasmids on an industrial scale;
- analytical separation of biomolecules;
- separation of undesired components such as viruses, proteases, cell debris, etc. from media which contain biomolecules for the purpose of pre-cleaning the media which contain biomolecules on an industrial scale;
- phytochemistry.

## Revendications

1. Procédé pour la séparation ou la purification de biomolécules par extraction aqueuse à deux phases au moyen d'une première phase aqueuse et d'une deuxième phase aqueuse,
dans lequel au moins un transfert partiel des biomolécules dans la deuxième phase aqueuse est effectué pendant la séparation,
dans lequel un matériau poreux est présent, dans les pores duquel la deuxième phase aqueuse est présente et pendant la séparation une solution de biomolécules est présente dans la deuxième phase aqueuse dans les pores du matériau poreux, et dans lequel le matériau poreux est un matériau en forme de particules
dans lequel pendant l'exécution de la séparation ou de la purification la deuxième phase aqueuse est essentiellement présente dans les pores du matériau poreux, ainsi que avant et après la séparation la première phase aqueuse se trouve essentiellement à l'extérieur du matériau poreux.
et dans lequel les biomolécules sont sélectionnées parmi des acides nucléiques, des antibiotiques, des aminoacides, des lipides, des glucides, des métabolites, des corps d'inclusion, des produits métaboliques et toutes sortes de protéines.

2. Procédé selon la revendication 2, dans lequel la taille moyenne des pores du matériau poreux s'élève de ≥ 0,1 à ≤ 5000 nm

3. Procédé selon la revendication 1 ou la revendication 2, dans lequel la taille moyenne des particules du matériau poreux s'élève de ≥ 0,01 mm à ≤ 20 mm

4. Procédé selon l'une quelconque des revendications 1 à 3, dans lequel le matériau poreux présente un taux de gonflement de ≤ 40%

5. Procédé selon l'une quelconque des revendications 1 à 4, dans lequel le matériau poreux est essentiellement sélectionné parmi le groupe contenant
- des particules de polymère à base de polystyrène-divinylbenzène, polypropylène, mélamine, poly(méthacrylate de méthyle), acétate de polyvinyle, chlorure de polyvinyle, polystyrène, polyacrylamide, acrylate de butyle; ces polymères étant éventuellement sous forme amorphe, semi-cristalline et cristalline
- des particules (polymériques) magnétiques
- des microcapsules
- des particules de polymères carbonées, aussi appelées résines carbonées
- des particules de polymère revêtues, aussi appelées coated particles, dans lequel le revêtement peut être réalisé entre autres au moyen d'un alcool de polyvinyle
- des anions polymères et échangeurs de cations
- du zéolite
- de la silice / du silicate
- du charbon actif
- des anions inorganiques et des échangeurs de cations
- des structures organométalliques
- du verre
- du carbonate
- des mousses de polymère
- des mousses métalliques
ou des mélanges de ceux-ci.

6. Utilisation d'un procédé selon l'une des revendications 1 à 5 pour
- la production à grande échelle de protéines
- la production à grande échelle d'antibiotiques, d'aminoacides, d'(oligo-)peptides, d'acides nucléiques, de lipides, de glucides, de métabolites, de produits métaboliques, de corps d'inclusion, de plasmides
- la séparation analytique de biomolécules
- la séparation à grande échelle des composants indésirables tels que des virus, des protéases, des débris cellulaires, etc. à partir de médias contenant des biomolécules en vue du pré-nettoyage des médias contenant les biomolécules
- la phytochimie
